# EUROPEAN PATENT APPLICATION

(11) **EP 3 513 767 A1**
(43) Date of publication of application: **24.07.2019**
(21) Application number: 18152410.9
(22) Date of filing: 18.01.2018
(51) Int. Cl.: A61D 1/02, A61K 9/00, A61K 33/24

(54) **INTERNAL TEAT SEALANTS AND THEIR USE IN THE PREVENTION OF BOVINE MASTITIS IN THE DRY COW**

(71) Applicant: BIMEDA RESEARCH & DEVELOPMENT LIMITED, D18 K8Y4 Dublin 18 (IE)
(72) Inventor: SMITH, Brendan Gerard, Dublin, D03 V9F7 (IE)
(74) Representative: O'Brien, John Augustine

(57) **Abstract**

An intramammary syringe contains a seal formulation for forming a physical barrier in the teat canal of a non-human animal. The syringe contains a reduced volume of from 0.25ml to 2.0ml of the seal formulation. The problem of ascending sealant during the dry period is solved.

## Description

### Introduction

All dairy cows require a period of time prior to calving, where milk production is stopped, in order to prepare for the next lactation and allow the mammary tissues repair and regenerate. Typically, the duration of this period is between 40 and 70 days.

In modern dairy farming, the process of stopping the milk production is known as drying off and can be achieved by a number of methods, including reduction of diet and water; introduction of biological response modifiers (often hormonal); and the withdrawal of stimuli - particularly the cessation of the regular milking routine.

Once the decision to dry off has been taken and the cow is no longer being milked, the cow's system commences a sequence known as involution. The cow is now in what is commonly referred to as the dry period.

During the dry period, the cow is very susceptible to the ingress of bacteria via the teat canal, which can very often remain open for a considerable time (in some cases never closing throughout the dry period). In a 1995 study in New Zealand, Williamson et al., found that 50% of teats were still open 10 days after drying off (NZ Vet. J. 43 (6) 228-34). More recent studies on higher yielding cows showed that, after 6 weeks of observation, 23.4% of teats remained open (Dingwell et al. Prev. Vet. Med. 2004 April 30, 63(1-2):75-89).

Where a teat is open during the dry period, bacteria can invade the udder where they can cause infection, known as mastitis. Due to the presence of high levels of lactoferrin (a protein that binds up available iron) in the udder during the dry period, many of the bacteria will not be able to multiply at this time. However, 50 - 60% of all new infections caused by environmental pathogens occur during the dry period (Bradley and Green, J. Dairy Sci., 2000, Sept; 83(9): 1957-65) and over 50% of clinical coliform mastitis events in the first 100 days in milk originated from bacteria that entered the udder during the dry period (Bradley and Green, J. Dairy Sci., 2002, Mar; 85(3):551-61).

Accordingly, it is highly desirable to prevent the ingress of bacteria during the dry period and thus prevent clinical mastitis in the next lactation.

The use of internal teat sealants at drying off, is designed to provide a sustained physical barrier in the teat cistern, thereby preventing the ingress of bacteria into the udder. This is a well-established practice in dairy husbandry, with a number of products licensed globally for this purpose.

In developed dairy markets, teat sealants are used on many dairy cows and heifers, with usage levels of above 50% not uncommon. In many markets, teat sealants are used in conjunction with a long acting antibiotic intramammary infusion, which is administered immediately prior to the teat sealant. The antibiotic intramammary infusion is massaged up into the udder, whereas the teat sealant is maintained in the streak canal / teat cistern.

In many cases, the use of an antibiotic at drying off is unjustified and with the global concerns relating to antimicrobial resistance, this practice is now being discontinued in many countries and is only permitted where it has been determined that there is a pre-existing subclinical mastitic infection.

In many of the developed markets, up to 70% of dairy cows are free of such subclinical infections at drying off and do not require any antibiotic treatment.

As the use of antibiotics at drying off declines, the use of internal teat sealants, to prevent the ingress of bacteria during the dry period, has grown substantially in recent years.

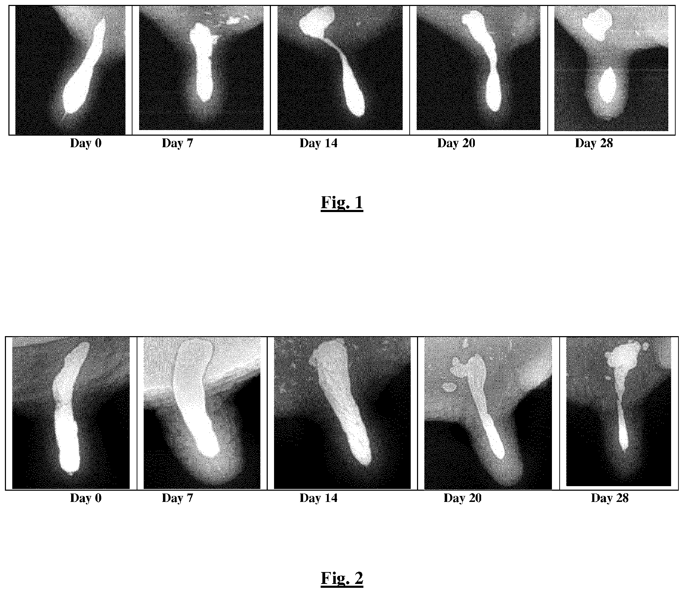

Typically, teat sealants comprise 65% w/w bismuth subnitrate (a non-toxic heavy metal salt) in a gel base. A 4g infusion of the sealant is administered to each teat. The sealant is intended to remain cohesive within the teat during the dry period. However, we have found that in some cases, as the dry period progresses, the three dimensional structure of the sealant can change and in some cases break into separate pieces. Figs. 1 and 2 are images from an x-ray study of teats during a 28 day dry period. In the day 28 images it will be noted that an upper fraction of the seal has ascended from the teat cistern into the gland cistern. In some cases the entire structure remains intact, but moves up into the gland cistern, only to descend again in the later stages of the dry-period.

In some cases particles of internal teat sealants that have become bound to the mammary tissues in the udder and will stay there up to and sometimes for some days post calving. Where this occurs, it is often only when the cow has been milked on a number of occasions that these particles will detach from the mammary tissues and will become mixed with the milk. In instances such as this, the particles of internal teat sealant are known to either lodge in the milk lines or on occasions can make it through to the milk collection point.

### Statements of Invention

According to the invention there is provided an intramammary syringe containing a seal formulation for forming a physical barrier in the teat canal of a non-human animal wherein the syringe contains from 0.25ml to 2.0ml of the seal formulation.

The reduced volume of the seal formulation is sufficient to form an effective seal whilst avoiding the risk of sealant being forced upwardly as a result of the shrinkage of the udder anatomy during part of the dry period.

In one embodiment the syringe contains from 0.5ml to 2.0ml of the seal formulation, from 0.75ml to 1.75ml of the seal formulation, or from 1.0ml to 1.5ml of the seal formulation.

The invention also provides an intramammary syringe containing a seal formulation for forming a physical barrier in the teat canal of a non-human animal wherein the syringe contains from 0.5g to 2.5g of the seal formulation.

In one embodiment the syringe contains from 1.0g to 2.5g of the seal formulations.

In some cases the weight of the seal formulation contained in the syringe is from 1.5g to 2.0g.

In some embodiments the seal formulation comprises a heavy metal salt in a base.

In one case the heavy metal salt is bismuth subnitrate.

The bismuth subnitrate in some cases comprises approximately 65% wt of the seal formulation.

Other examples of non-toxic heavy metal salts include zinc oxide, barium sulphate and titanium dioxide. The seal formulation may comprise a number of such heavy metal salts.

The seal formulation may comprise a thixotrophic agent. The seal formulation in some cases contains from 0.1% to 1.5% of the thixotrophic agent, from 0.6 to 1.0% of the thixotrophic agent, or approximately 0.8% of the thixotrophic agent.

In some embodiments the thixotrophic agent comprises colloidal anhydrous silica.

In some embodiments the base is a gel based on aluminium stearate. The base may include liquid paraffin as a vehicle.

The invention also provides a method for forming a seal in the mammary gland of a non-human animal comprising the step of injecting through the teat canal from 0.25ml to 2.0ml of a seal formulation into the teat cistern.

In some embodiments the method comprises injecting through the teat canal from 0.5ml to 2.0ml of a seal formulation, or from 0.75ml to 1.75ml of a seal formulation.

In some embodiments the method comprises injecting through the teat canal from 1.0ml to 1.5ml of the seal formulation.

The formulation includes a thixotrophic agent or rheology modifier or emulsifier. One such is fumed silica which is also known as anhydrous colloidal silica. It is available from Evonik under the Trade Name Aerosil. It is also available from Cabot Corporation (Cab-o-sil) and Wacker Chemie - Owens Corning and OCI (Konasil).

The invention provides a teat seal which provides an effective physical barrier to the teat canal of cattle for the prevention of intramammary infections throughout the dry period.

The teat seal of the invention has the following properties
- Non-toxic, biocompatible, and capable of being sterilised.
- Persistent - the seal remains *in situ* for the duration of the dry cow period
- Consistency - the seal does not break up within the teat
- Ease of removal- at the end of the dry period the seal is easily removable from the udder and does not give rise to persistent residues of the seal
- Radiopaque
- Ease of delivery

Preferably, the teat seal formulation does not have antibiotic or anti-infective properties. The seal should not contain an antibiotic. In some cases the seal does not contain a plant oil such as thyme oil.

### Brief Description of the Drawings

The invention will be more clearly understood from the following description thereof, given by way of example only, in which:-
Figs. 1 and 2 are x-ray studies of teats into which an internal teat sealant has been infused.

### Detailed Description

When cows are dried off, a process known as involution occurs within the udder. Involution has three distinct phases.

### 1) Active Involution

The first of these stages is known as active involution and, depending on the time remaining to calving (and consequently the length of the dry-period), this will usually be complete within 21 to 30 days. During this time the udder more or less maintains it's pre-dry off state, with milk continuing to accumulate for approximately 4 days and then declines rapidly over the next week. Fluid volume continues to decrease through -30 days (in a 45-60 day dry-period).

### 2) Steady State Involution

As the volume of fluid reduces, the udder shrinks considerably (upwards and inwards) in size. The cow is now in steady state involution.

We have discovered that as the udder shrinks, during this state, so does the available area within the teat canal, sometimes leaving an area inadequate to accommodate the seal volume. Such is the pressure within the shrinking teat cistern that the internal teat sealant can be torn apart, with portions of it being forced up into the udder. It has been observed that the teat sealant plug, which has earlier formed in the teat cistern, can in some cases be forced upwards in its entirety into the gland cistern.

The length of the steady state period depends on the total length of the dry period. If active involution takes in the region of 4 weeks to complete in the dairy cow and the redevelopment stage takes about 3 or 4 weeks. These periods will then account for the recommended optimal 45-60 day dry period. Accordingly, cows with a 45-60 day dry period probably have a very short steady state phase, or no steady state phase of involution, at all. In instances where no steady state occurs, there is little or no pressure on the internal teat sealant.

### 3) Colostrogenesis

During the third phase of the dry-period, known as colostrogenesis and lactogenesis, the udder and teats begin to expand again.

This phase of the dry period marks the transition from the non-lactating state to the lactating state. It is not known exactly when this period begins, but it usually occurs around 3 to 4 weeks before calving occurs.

During this time the volume of the teat canal increases, often allowing non-bound particles of internal teat sealant that have been forced up into the udder during steady state involution, settle back into the teat cistern. However, studies have also shown that some particles of internal teat sealants that have been forced upwards become bound to the mammary tissues in the udder and will stay up there and sometimes for some days post calving. Where this occurs, it is often only when the cow has been milked on a number of occasions that these particles will detach from the mammary tissues and will become mixed with the milk. In instances such as this, the particles of internal teat sealant are known to either lodge in the milk lines or on occasions can make it through to the milk collection point.

We have surprisingly discovered that when a smaller volume of teat sealant is initially infused the likelihood of it being forced upwards from the teat cistern into the gland cistern is significantly reduced, or eliminated entirely.

### Example 1 - Teat Seal Formulation

| **Component** | **Quantity per g** | **Quantity (% w/w)** |
|---|---|---|
| **Bismuth Subnitrate** | 650.0 mg | 65 % |
| **Colloidal Anhydrous silica** | 8.0 mg | 0.8 % |
| **Aluminium di/tri stearate** | 48.0 mg | 4.8 % |
| **Liquid paraffin, Heavy** | q.s. 1 g | q.s. 100 % |

The formulation above was prepared by the following process:
Liquid paraffin, heavy is added to a vessel.
Aluminium di-/tri stearate is added to the liquid paraffin, heavy, stirred and heated to a minimum of 150°C.
The mixture is maintained at this temperature for a minimum of 3 hours.
The mixture is cooled and the Bismuth Subnitrate and Colloidal Anhydrous Silica is then added and mixed until homogenous.
The product is then filled into intramammary syringes. The amount filled into the syringe is from 0.5 to 2.0 ml or 1.0g to 2.5g to be administered as a single dose.
The filled syringes may be sterilised by gamma irradiation.

### Example 2 - Use of the Teat Seal Formulation

Studies have shown that by reducing the conventionally accepted dose of 4g per teat at drying off, down to between 0.5g and 2.5g, the effectiveness of the internal teat sealant is not diminished, but that the problem of ascending internal teat sealant during involution is eliminated.

The invention is not limited to the embodiments hereinbefore described, which may be varied in detail.

## Claims

1. An intramammary syringe containing a seal formulation comprising a heavy metal salt in a gel base for forming a physical barrier in the teat canal of a non-human animal wherein the syringe contains from 0.25ml to 2.0ml of the seal formulation.

2. An intramammary syringe as claimed in claim 1 wherein the syringe contains from 0.5ml to 2.0ml of the seal formulation.

3. An intramammary syringe as claimed in claim 1 or 2 wherein the syringe contains from 0.75ml to 1.75ml of the seal formulation.

4. An intramammary syringe as claimed in any of claims 1 to 3 wherein the syringe contains from 1.0ml to 1.5ml of the seal formulation.

5. An intramammary syringe containing a seal formulation comprising a heavy metal salt in a gel base for forming a physical barrier in the teat canal of a non-human animal wherein the syringe contains from 0.5g to 2.5g of the seal formulation.

6. An intramammary syringe as claimed in claim 5 wherein the syringe contains from 1.0g to 2.5g of the seal formulation.

7. An intramammary syringe as claimed in claim 5 or 6 wherein the weight of the seal formulation contained in the syringe is from 1.5g to 2.0g.

8. An intramammary syringe as claimed in any of claims 1 to 7 wherein the heavy metal salt is bismuth subnitrate.

9. An intramammary syringe as claimed in claim 8 wherein the bismuth subnitrate comprises approximately 65% wt of the seal formulation.

10. An intramammary syringe as claimed in any of claims 1 to 9 wherein the seal formulation comprises a thixotrophic agent.

11. An intramammary syringe as claimed in claim 10 wherein the seal formulation contains from 0.1% to 1.5% of the thixotrophic agent, from 0.6 to 1.0% of the thixotrophic agent, or approximately 0.8% of the thixotrophic agent.

12. An intramammary syringe as claimed in claim 10 or 11 wherein the thixotrophic agent comprises colloidal anhydrous silica.

13. An intramammary syringe as claimed in any of claims 1 to 12 wherein the base is a gel based on aluminium stearate, optionally the base includes liquid paraffin as a vehicle.

14. A method for forming a seal in the mammary gland of a non-human animal comprising the step of injecting through the teat canal from 0.25ml to 2.0ml of a seal formulation comprising a heavy metal salt in a gel base into the teat cistern.

15. A method as claimed in claim 18 comprising injecting through the teat canal from 0.5ml to 2.0ml of the seal formulation, from 0.75ml to 1.75ml of the seal formulation, or from 1.0ml to 1.5ml of the seal formulation.
